# EUROPEAN PATENT SPECIFICATION

(11) **EP 1 732 577 B1**
(45) Date of publication and mention of the grant of the patent: **10.04.2013**
(21) Application number: 05723558.2
(22) Date of filing: 23.02.2005
(51) Int. Cl.: A61K 8/20, A61K 8/44, A61Q 19/08

(54) **TOPICAL DELIVERY OF L-ARGININE TO IMPROVE BODY AND SKIN APPEARANCE**
TOPISCHE VERABREICHUNG VON L-ARGININ ZUR VERBESSERUNG DES AUSSEHENS VON KÖRPER UND HAUT
ADMINISTRATION TOPIQUE DE L-ARGININE, DESTINEE A AMELIORER L'ASPECT DU CORPS ET DE LA PEAU

(30) Priority: 23.02.2004 US 546214 P; 19.04.2004 US 563566 P
(43) Date of publication of application: 20.12.2006
(73) Proprietor: Strategic Science & Technologies, LLC, Cambridge MA 02141 (US)
(72) Inventor: FOSSEL, Eric, Thor, Cambridge MA 02139 (US)
(74) Representative: Jump, Timothy John Simon
(86) International application number: PCT/US2005/005726
(87) International publication number: WO 2005/081964

(56) References cited:
- EP-A- 1 210 933
- WO-A-00/03689
- WO-A-00/40215
- WO-A-88/06034
- WO-A-99/13717
- DE-A1- 10 128 910
- FR-A- 1 553 063
- FR-A- 2 810 540
- US-A- 5 254 331
- US-A- 5 476 852
- US-A- 5 538 740
- US-A1- 2002 168 325
- SCHOLERMANN A ET AL: "Clinical and biophysical efficacy of a novel body cream (Eucerin<(>R) amino body cream) for aged dry skin containing urea and L-arginine" JEADV. JOURNAL OF THE EUROPEAN ACADEMY OF DERMATOLOGY AND VENEREOLOGY, ELSEVIER SCIENCE PUBLISHERS, AMSTERDAM, NL, vol. 11, 1 September 1998 (1998-09-01), page S270, XP004556076 ISSN: 0926-9959

## Description

### FIELD OF INVENTION

This invention generally relates to methods and compositions for improving body and skin appearance.

### BACKGROUND

There have been many approaches to improving body and skin appearance, using both systemic and topical approaches. One method of tightening skin to improve appearance is through the use of cosmetic surgery. For instance, for sagging skin or larger wrinkles for double chins, an individual may resort to cosmetic surgery, for example, a facelift or a tuck. Sagging breasts have also been treated surgically. However, the problems associated with this approach are obvious with the high cost and the risks associated with undergoing any medically unnecessary surgery.

Another surgical method to improve skin appearance of areas of the skin, such as the chins and arms, is through liposuction. Liposuction is effective for improving the appearance of skin, but it has a very high cost and there can be side effects, such as infections that can lead to death.

Radiofrequency energy is another method increasingly being used to tighten skin without the need for surgery, such as in a conventional facelift, reducing some of the potential surgical risks such as infection and anesthesia. This medical procedure is still troublesome to many individuals, however, because it can cause damage to underlying tissues.

Treatments of the skin without the use of cosmetic surgery include many different techniques, but there are relatively few treatments that are effective in providing any noticeable benefits relative to the prohibitive costs. For instance, a popular method of providing the appearance of a tightening of the skin is to remove small wrinkles through the use of alpha lipoic acid. This treatment does not cause much of a tightening effect, only the removal of time wrinkles, thereby providing the appearance of tightening. Although many treatments can produce a negative reaction in some people, adverse reactions to lipoic acid are somewhat less common than to agents such as RetinA, vitamin C or glycolic acid. Alpha lipoic acid is useful in treating small wrinkles but it can result in rashes, because of reaction to the acid.

### SUMMARY OF THE INVENTION

This invention generally relates to improvement of the body and skin appearance. The subject matter of the present invention involves, in some cases, interrelated products, alternative solutions to a particular problem, and/or a plurality of different uses of one or more systems and/or articles.

The present invention provides, in a first aspect, a cosmetic method comprising an act of applying a delivery vehicle comprising L-arginine to a breast, for a period of time sufficient to reduce sagging in the breast, wherein the delivery vehicle comprises a penetrating agent comprising an ionic salt present at a concentration of at least 5 wt %.

In a second aspect, the invention provides a cosmetic method comprising an act of applying a delivery vehicle containing L-arginine to a breast, for a period of time sufficient to allow the breast to absorb a sufficient quantity of L-arginine to produce a smoother surface on the breast, wherein the delivery vehicle comprises a penetrating agent comprising an ionic salt present at a concentration of at least 5 wt %.

The instant invention provides beneficial effects in the appearance of the body and skin, for instance by smoothing skin that is wrinkled, sagging, or cellulite-afflicted. In one set of embodiments, the application of a delivery vehicle such as a cream, liquid, lotion, spray, aerosol, or transdermal patch containing L-arginine in a sufficient concentration to improve the appearance of a selected area of the body may be applied.

For example, a person with breast ptosis (e.g., pseudoptosis, partial ptosis, or true ptosis) may be treated using an embodiment of the invention.

In an embodiment of the invention, a cream containing L-arginine at an effective concentration, may create a hostile biophysical environment that facilitates absorption of the L-arginine into the skin.

In some cases, the instant invention may be used to enhance the appearance of the body using the body's natural mechanisms. For example, the instant invention may be used to remove small wrinkles, or to tighten sagging breasts.

Other advantages and novel features of the present invention will become apparent from the following detailed description of various non-limiting embodiments of the invention when considered in conjunction with the accompanying figures.

### BRIEF DESCRIPTION OF THE DRAWINGS

Non-limiting embodiments of the present invention will be described by way of example with reference to the accompanying figures. In the figures:
Figs. 1A-1B illustrate the use of an embodiment of the invention to treat a person's breasts; and
Figs. 2A-2B illustrate the use of another embodiment of the invention in the treatment of a person's breasts.

### DETAILED DESCRIPTION

This invention generally relates to improvement of the body and skin appearance, for example enhancing the appearance of sagging, wrinkled, or cellulite-afflicted areas of the skin and body, through the local delivery of L-arginine, a nitric oxide donor, for example, using delivery vehicles such as lotions, creams, liquids, sprays, aerosols, and/or transdermal patches. In some embodiments, a delivery vehicle containing L-arginine (an important biological precursor) in a sufficient concentration to improve the appearance of a selected area of the body may be applied. One or more penetrating agents are included that aid in the transfer of the L-arginine into the tissue, which may overcome the resistance to transfer into the skin. Non-limiting examples of suitable penetrating agents include, choline chloride, magnesium chloride, and/or sodium chloride.

In one aspect, the topical application of the nitric oxide donor L-arginine may be used to cause a beneficial effect to the area of the skin applied, for example, a cosmetic effect, such as improving body or skin appearance. Nitric oxide may cause changes in the skin through natural biological responses that react to the presence of nitric oxide. For example, in response to the presence of nitric oxide, the skin may smoothen, tighten, or become more firm (i.e., the viscoelasticity of the skin may increase), which may result in an improvement in appearance. In some cases, a Greast having a sagging appearance may be treated using various embodiments of the invention. For example, the sagging appearance of the breasts may be reduced; for instance, a breast may appear fuller and/or raised after treatment. As a particular example, a person with breast ptosis (e.g., pseudoptosis, partial ptosis, or true ptosis) may be treated using an embodiment of the invention. In some cases, the topical application of L-arginine may improve appearance by causing an increase in tissue volume, which may result in increased size or firmness, and/or decrease sagging. In certain instances, the improved appearance may be measured, for example, by measuring a change in the viscoelasticity of the skin.

In some embodiments, L-arginine may be administered using a delivery vehicle such as a cream, liquid, lotion, spray, aerosol, or transdermal patch. Examples of delivery vehicles are discussed below. The delivery vehicle may promote transfer into the skin of an effective concentration of nitric oxide, directly or indirectly, through L-arginine penetrating into at least a portion of the skin. For instance, the delivery vehicle includes one or more penetrating agents, as further described herein. In some embodiments, the delivery vehicle may include a hostile biophysical environment, e.g., using a penetrating agent alone or in combination with other agents, as further discussed herein.

In certain embodiments of the invention, multiple treatments of the delivery vehicle may increase the duration of the effects of nitric oxide, for example two, three, four, five, or more treatments may be applied, depending on the particular application. For example, with repeated administrations, the beneficial effects of each treatment may be extended up to ten or twenty hours after treatment, or more in some cases. In certain cases, the concentration of L-arginine can be reduced after the initial treatment to maintain the same desired duration of cosmetic effect. Such treatments may be given at any suitable frequency, depending on the particular application, for example, every 4 hours, every 8 hours, every 12 hours, every 18 hours, every 1 day, every 2 days, every 3 days, every week, etc. For instance, the treatment may be provided between about 2 and about 30 times within a time period of about 30 days. In some cases, the first treatment may be given at a higher level or concentration than subsequent treatments.

A "nitric oxide donor," as used herein, is a compound that contains a nitric oxide moiety, where the compound is able to release nitric oxide and/or chemically transfer the nitric oxide moiety to another molecule, directly or indirectly, for example, through a biological process. The nitric oxide donor may release nitric oxide into the skin, and/or tissues such as muscles and/or elements of the circulatory system in close proximity to the surface of the skin. The nitric oxide donor used in the present invention is L-arginin. In some cases, the concentration of L-arginine may be tailored to have a duration of effective treatment of at least about 3 hours, at least about 5 hours, or at least about 8 hours or more in certain instances. The duration may also be controlled, for instance, by controlling the concentration of the penetrating agent used in conjunction with the L-arginine. The actual concentration for a particular application can be determined by those of ordinary skill in the art using no more than routine experimentation, for example, by measuring the amount of transport of nitric oxide and/or L-arginine donor as a function of concentration *in vitro* across cadaver skin or suitable animal models, skin grafts, synthetic model membranes, or the like.

As a particular non-limiting example, in one embodiment, nitric oxide is provided using L-arginine, for example, at a concentration of at least about 0.50% by weight (wt% or w/v) of L-arginine (with one or more penetrating agents as discussed herein ), at least about 0.75 wt%, at least about 1 wt%, at least about 2 wt%, at least about 3 wt%, at least about 5 wt%, at least about 7 wt%, at least about 10 wt%, or at least about 15 wt%. The L-arginine may be present in a suitable delivery vehicle, such as a cream or a lotion. L-arginine may be particularly useful in some cases due to its low toxicity, its high solubility, or its low cost.

Examples of the ionic salt include sodium chloride, magnesium chloride, calcium chloride, and/or choline chloride. The ionic salt(s) is at a concentration sufficient to aid in tissue absorption of nitric oxide and/or L-arginine. As another example, the L-arginine may also be used in conjunction with an adjunct, such as theophylline. Other examples of penetrating agents include, but are not limited to, cationic, anionic, or nonionic surfactants (e.g., sodium dodecyl sulfate, polyoxamers, etc.); fatty acids and alcohols (e.g., ethanol, oleic acid, lauric acid, liposomes, etc.); anticholinergic agents (e.g., benzilonium bromide, oxyphenonium bromide); alkanones (e.g., *n*-heptane); amides (e.g., urea, *N*,*N*-dimethyl-*m*-toluatnide); fatty acid esters (e.g., *n*-butyrate); organic acids (e.g., citric acid); polyols (e.g., ethylene glycol, glycerol); sulfoxides (e.g., dimethylsulfoxide); or terpenes (e.g., cyclohexene).

As discussed, in some embodiments, the L-arginine may be contained in a delivery vehicle such as a cream, liquid, lotion, spray, aerosol, or transdermal patch (which may contain a cream, liquid, lotion, spray, aerosol, or other formulation that allows transport of L-arginine to occur). Those of ordinary skill in the art will know of systems and techniques for incorporating bioactive compounds within delivery vehicles such as a cream, liquid, lotion, spray, aerosol, or transdermal patch. For example, the concentration of L-arginine within a delivery vehicle such as a cream or lotion may be at least about 0.1% w/v, between about 0.1 to 25% w/v, between about 5% w/v and 25% w/v, between about 10% w/v and 25% w/v, etc. In some cases, the concentration of L-arginine or in the delivery vehicle can be reduced with the inclusion of a greater amount or concentration of penetrating agent, or increased to lengthen the beneficial effect.

Thus, as one particular example, a delivery vehicle such as a cream or lotion may contain L-arginine hydrochloride with at least 12.5% weight by volume, combined with penetrating agents such as choline chloride having at least 10% weight by volume, sodium chloride with at least 5% weight by volume, and/or magnesium chloride with at least 5% weight by volume. In some cases, an adjunct such as theophylline may also be used (for example, at 10% weight by volume). If a cream is used, other materials may be present within the cream, for example, buffers, preservatives, surfactants, etc. For instance, the cream may include one or more of water, mineral oil, glyceryl stereate, squalene, propylene glycol stearate, wheat germ oil, glyceryl stearate, isopropyl myristate, steryl stearate, polysorbate 60, propylene glycol, oleic acid, tocopherol acetate, collagen, sorbitan stearate, vitamin A and D, triethanolamine, methylparaben, aloe vera extract, imidazolidinyl urea, propylparaben, PND, or BHA.

As specific non-limiting examples, the cream may have one or more of (w/v): water (20-80%), white oil (3-18%), glyceryl stearate (0.25-12%), squalene (0.25-12%), cetyl alcohol (0.1-11%), propylene glycol stearate (0.1-11%), wheat germ oil (0.1-6%), polysorbate 60 (0.1-5%), propylene glycol (0.05-5%), collagen (0.05-5%), sorbitan stearate (0.05-5%), vitamin A (0.02-4%), vitamin D (0.02-4%), vitamin E (0.02-4%), triethanolamine (0.01-4%), methylparaben (0.01-4%), aloe vera extract (0/01-4%), imidazolidinyl urea (0.01-4%), propylparaben (0.01-4%), BHA (0.01-4%), L-arginine Hydrochloride (0.25-25%), sodium chloride (0.25-25%), magnesium chloride (0.25-25%), and/or choline chloride (0.25-25%). In this example, choline chloride, sodium chloride and magnesium chloride provide a high ionic strength environment for the highly charged molecule, L-arginine. This high ionic strength environment is an example of a hostile biophysical environment for L-arginine. That is, the highly charged ionic strength is an unfavorable environment for the highly charged L-arginine making the L-arginine anxious to move to a more hospitable, less charged environment such as human tissue.

The L-arginine may be packaged in such a way that it is carried into tissue and/or its charge is neutralized by derivitization and/or by forming a neutral salt. Ionic such as lithium chloride, sodium chloride, potassium chloride, calcium chloride, magnesium chloride, choline chloride, sodium fluoride, lithium bromide, etc., as well as combinations of these and/or other salts, for instance at high ionic strengths, such as between about 0.25 M and about 15 M, between about 5 M and about 15 M, between about 10 M and about 15 M, etc.); can be-used in the present invention; as can high or low pH environments (e.g., by adding pharmaceutically acceptable acids or bases, for example, such that the pH is between about 3 and about 7, between about 3 and about 6, between about 3 and about 5, between about 7 and about 11, between about 8 and about 11, between about 9 and about 11, etc.); or highly hydrophobic environments (e.g., by decreasing water content and increasing lipid, oil and/or wax content of the environment). Other highly charged molecules such as polylysine, polyglutamine, polyaspartate, etc., or copolymers of such highly charged amino acids may also be used in certain embodiments. Non-limiting examples of packaging which would be carried into tissue includes liposomes or emulsions of collagen, collagen peptides or other components of skin or basement membrane. Non-limiting examples of neutralization of charge include delivery of the L-arginine in the form or an ester or salt which is electronically neutral. For example, L-arginine may be delivered as a neutral compound such as L-arginine glutamate.

In some embodiments the L-arginine is placed into a hydrophobic, oily environment such as in an oil-based cream or lotion containing little or no water. Absorption may further be aided by combining the use of hostile biophysical environments with the use of penetrating agents.

The following documents include rélement disclosures : U.S. Provisional Patent Application Serial No. 60/546,214, filed February 23, 2004, entitled "Topical Delivery of a Nitric Oxide Donor to Improve Body and Skin Appearance," by E.T. Fossel; U.S. Provisional Patent Application Serial No. 60/563,566, filed April 19, 2004, entitled "Transdermal Delivery of L-Arginine for the Purpose of Enhancing the Appearance of the Female Breast," by E.T. Fossel; U.S. Patent Application Serial No. 08/932,227, filed September 17,1997, entitled "Topical Delivery of Arginine of Cause Beneficial Effects," by E.T. Fossel, published as 2002/0041903 on April 11, 2002; U.S. Patent Application Serial No. 10/201,635, filed July 22, 2002, entitled "Topical Delivery of L-Arginine to Cause Beneficial Effects," by E.T. Fossel, published as 2003/0028169 on February 6, 2003; U.S. Patent Application Serial No. 10/213, 286, filed August 5, 2002, entitled "Topical and Oral Arginine to Cause Beneficial Effects," by E.T. Fossel, published as 2003/0018076 on January 23, 2003; International Patent Application No. PCT/US98/19429, filed September 17, 1998, entitled "A Delivery of Arginine to Cause Beneficial Effects," by E.T. Fossel, published as WO 99/13717 on March 25, 1999; U.S. Patent No. 5,895,658, issued April 20, 1999, entitled "Topical Delivery of L-Arginine to Cause Tissue Warming," by E.T. Fossel; U.S. Patent No. 5,922,332, issued July 13, 1999, entitled "Topical Delivery of Arginine to Overcome Pain," by E.T. Fossel; U.S. Patent No. 6,207,713, issued March 27, 2001, entitled "Topical and Oral Delivery of Arginine to Cause Beneficial Effects," by E.T. Fossel; and U.S. Patent No. 6,458,841, issued October 1, 2002, entitled "Topical and Oral Delivery of Arginine to Cause Beneficial Effects," by E.T. Fossel.

The following examples are intended to illustrate certain embodiments of the present invention, but do not exemplify the full scope of the invention.

### EXAMPLE 1

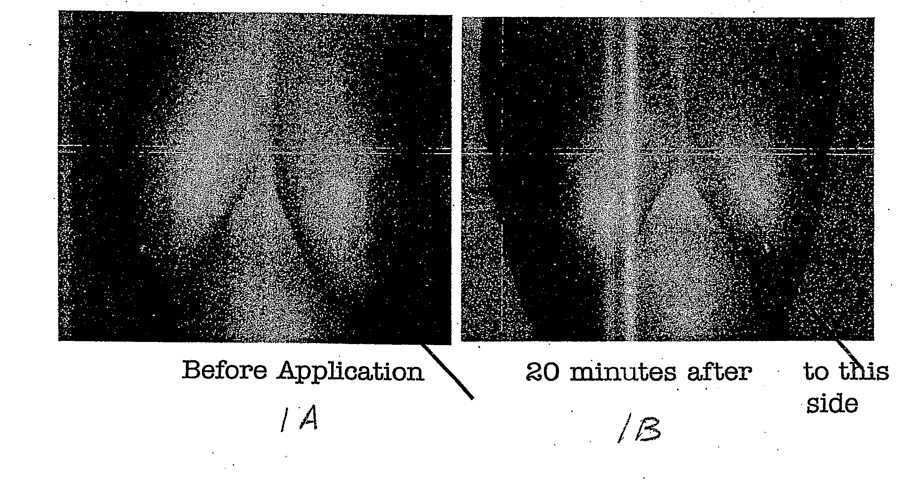

This example illustrates the reduction of breast sagging and an increase of breast firmness. In this example, a 60-year-old woman with pendulous breasts (Fig. 1A) was provided with a cream comprising L-arginine (12.5% w/v), sodium chloride (10% w/v), and magnesium chloride (5% w/v). The cream was applied to one of the breasts, which was rubbed in extensively for maximal absorption. After a period of approximately 20 minutes the treated breast was much fuller and raised up by about 1.5 inches (Fig. 1B). The effect of the initial treatment lasted for a period of about seven hours. The concentration of L-arginine could also be reduced to decrease the duration of the cosmetic effect of the initial application.

### EXAMPLE 2

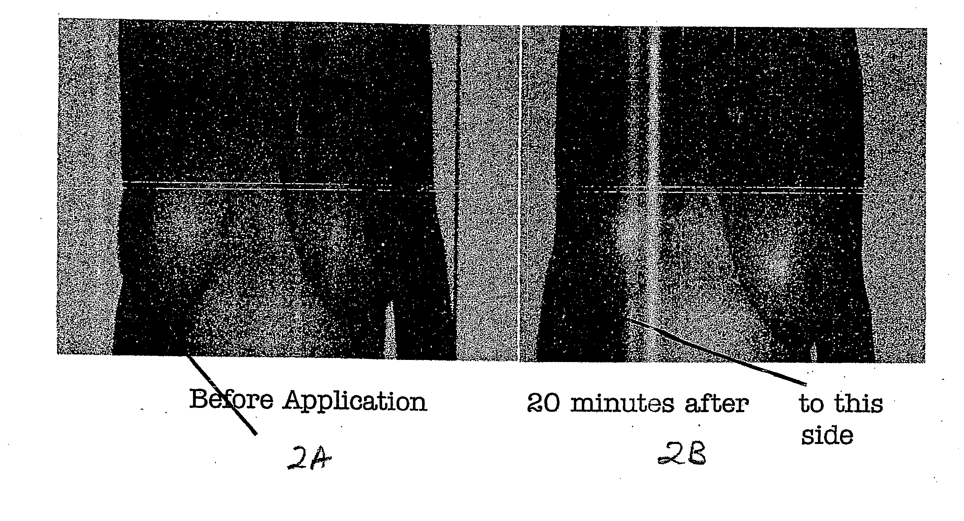

This example illustrates the reduction of breast sagging and an increase of breast firmness. In this example, a 47-year-old woman, with pendulous breasts (Fig. 2A) applied a breast lifting cream comprising L-arginine (12.5% w/v), choline chloride (10% w/v), sodium chloride (10% w/v), and magnesium chloride (5% w/v). The breast lifting cream was rubbed vigorously into each breast for about five minutes. Within one hour both breasts were noticeably firmer and had been lifted about 2.75 inches (Fig. 2B). The effect of the initial treatment lasted for about five hours.

The treatment was continued daily for about a month. The lifting effect of the treatment had an effective duration of about 18 to 20 hours after about a month of daily use. The concentration of L-arginine could also be maintained to continue cosmetic benefits for up to twenty hours if the same cream is applied on a regular basis of once every 8 to 48 hours, or every 12 to 36 hours.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims, the invention may be practiced otherwise than as specifically described and claimed. The present invention is directed to each individual feature, system, article, material, kit, and/or method described herein. In addition, any combination of two or more such features, systems, articles, materials, kits, and/or methods, if such features, systems, articles, materials, kites, and/or methods are not mutually inconsistent, is included within the scope of the present invention.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

## Claims

1. A cosmetic method comprising an act of applying a delivery vehicle comprising L-arginine to a breast, for a period of time sufficient to reduce sagging in the breast, wherein the delivery vehicle comprises a penetrating agent comprising an ionic salt present at a concentration of at least 5 wt %.

2. The method of claim 1, wherein the sagging is determined using viscoelasticity.

3. The method of claim 1, wherein the effective concentration of L-arginine is at least 5% by weight/volume of the delivery vehicle.

4. The method of claim 1, wherein the delivery vehicle further comprises one or more of water, mineral oil, glyceryl stereate, squalene, propylene glycol stearate, wheat germ oil, glyceryl stearate, isopropyl myristate, steryl stearate, polysorbate 60, propylene glycol, oleic acid, tocopherol acetate, collagen, sorbitan stearate, vitamin A, vitamin D, triethanolamine, methylparaben, aloe vera extract, imidazolidinyl urea, propylparaben, PND or BHA.

5. The method of claim 1, wherein the penetrating agent is present in the delivery vehicle at a concentration at least sufficient to allow the L-arginine to act for at least about 3 hours.

6. The method of claim 1, further comprising an act of reapplying the delivery vehicle to the breast, optionally comprising repeating the act of reapplying the delivery vehicle to the breast between 2 and 30 times, inclusively, within a time period of about 30 days.

7. A cosmetic method comprising an act of applying a delivery vehicle containing L-arginine to a breast, for a period of time sufficient to allow the breast to absorb a sufficient quantity of L-arginine to produce a smoother surface on the breast, wherein the delivery vehicle comprises a penetrating agent comprising an ionic salt present at a concentration of at least 5 wt %.

8. The method of claim 7, wherein the delivery vehicle comprises one or more of water, mineral oil, glyceryl stereate, squalene, propylene glycol stearate, wheat germ oil, glyceryl stearate, isopropyl myristate, steryl stearate, polysorbate 60, propylene glycol, oleic acid, tocopherol acetate, collagen, sorbitan stearate, vitamin A, vitamin D, triethanolamine, methylparaben, aloe vera extract, imidazolidinyl urea, propylparaben, PND or BHA.

9. The method of claim 7, further comprising an act of reapplying the delivery vehicle to the breast, optionally comprising repeating the act of reapplying the delivery vehicle to the breast after between about 8 hours and about 48 hours after the act of applying the delivery vehicle.

10. The method of claim 1 or 7, wherein the delivery vehicle is a cream.

11. The method of claim 1 or 7, comprising rubbing the delivery vehicle into the breast.

12. The method of claim 1 or 7, wherein the ionic salt comprises one or more of lithium chloride, sodium chloride, potassium chloride, calcium chloride, magnesium chloride, or choline chloride.

13. The method of claim 1 or 7, wherein the ionic salt is present at a concentration of at least about 10% by weight.

## Patentansprüche

1. Kosmetisches Verfahren, das eine Handlung der Auftragung eines Abgabevehikels, das L-Arginin umfasst, auf eine Brust für eine Zeitdauer umfasst, die ausreicht, um die Erschlaffung in der Brust zu verringern, worin das Abgabevehikel ein eindringendes Mittel umfasst, das ein ionisches Salz umfasst, das in einer Konzentration von mindestens 5 Gew.-% vorliegt.

2. Verfahren nach Anspruch 1, worin die Erschlaffung unter Verwendung von Viskoelastizität bestimmt wird.

3. Verfahren nach Anspruch 1, worin die wirksame Konzentration von L-Arginin mindestens 5 Gewichts-%/Volumen des Abgabevehikels beträgt.

4. Verfahren nach Anspruch 1, worin das Abgabevehikel weiter eines oder mehrere von Folgenden umfasst: Wasser, Mineralöl, Glycerylstearat, Squalen, Propylenglycolstearat, Weizenkeimöl, Glycerylstearat, Isopropylmyristat, Stearylstearat, Polysorbat 60, Propylenglycol, Ölsäure, Tocopherolacetat, Kollagen, Sorbitanstearat, Vitamin A, Vitamin D, Triethanolamin, Methylparaben, Aloe-vera-Extrakt, Imidazolidinylharnstoff, Propylparaben, PND oder BHA.

5. Verfahren nach Anspruch 1, worin das eindringende Mittel im Abgabevehikel in einer Konzentration vorliegt, die zumindest ausreicht, um die Wirkung des L-Arginins für mindestens etwa 3 Stunden zu ermöglichen.

6. Verfahren nach Anspruch 1, das weiter eine Handlung der erneuten Auftragung des Abgabevehikels auf die Brust umfasst, wobei es optional die Wiederholung der Handlung der erneuten Auftragung des Abgabevehikels auf die Brust zwischen einschließlich 2- und 30-mal innerhalb einer Zeitdauer von etwa 30 Tagen umfasst.

7. Kosmetisches Verfahren, das eine Handlung der Auftragung eines Abgabevehikels, das L-Arginin enthält, auf eine Brust für eine Zeitdauer umfasst, die ausreicht, um die Aufnahme einer ausreichenden Menge von L-Arginin von der Brust zu ermöglichen, um eine glattere Oberfläche auf der Brust zu erzeugen, worin das Abgabevehikel ein eindringendes Mittel umfasst, das ein ionisches Salz umfasst, das in einer Konzentration von mindestens 5 Gew.-% vorliegt.

8. Verfahren nach Anspruch 7, worin das Abgabevehikel eines oder mehrere von Folgenden umfasst: Wasser, Mineralöl, Glycerylstearat, Squalen, Propylenglycolstearat, Weizenkeimöl, Glycerylstearat, Isopropylmyristat, Stearylstearat, Polysorbat 60, Propylenglycol, Ölsäure, Tocopherolacetat, Kollagen, Sorbitanstearat, Vitamin A, Vitamin D, Triethanolamin, Methylparaben, Aloe-vera-Extrakt, Imidazolidinylharnstoff, Propylparaben, PND oder BHA.

9. Verfahren nach Anspruch 7, das weiter eine Handlung der erneuten Auftragung des Abgabevehikels auf die Brust umfasst, wobei es optional die Wiederholung der Handlung der erneuten Auftragung des Abgabevehikels auf die Brust nach zwischen etwa 8 Stunden und etwa 48 Stunden nach der Handlung der Auftragung des Abgabevehikels umfasst.

10. Verfahren nach Anspruch 1 oder 7, worin das Abgabevehikel eine Creme ist.

11. Verfahren nach Anspruch 1 oder 7, das das Einreiben des Abgabevehikels in die Brust umfasst.

12. Verfahren nach Anspruch 1 oder 7, worin das ionische Salz eines oder mehrere von Folgenden umfasst: Lithiumchlorid, Natriumchlorid, Kaliumchlorid, Calciumchlorid, Magnesiumchlorid oder Cholinchlorid.

13. Verfahren nach Anspruch 1 oder 7, worin das ionische Salz in einer Konzentration von mindestens etwa 10 Gewichts-% vorliegt.

## Revendications

1. Procédé cosmétique comprenant un acte consistant à appliquer un excipient d'administration comprenant de la L-arginine sur un sein, pendant une période de temps suffisante pour réduire le relâchement du sein, dans lequel l'excipient d'administration comprend un agent de pénétration comprenant un sel ionique présent dans une concentration d'au moins 5 % en poids.

2. Procédé selon la revendication 1, dans lequel le relâchement est déterminé à l'aide de la viscoélasticité.

3. Procédé selon la revendication 1, dans lequel la concentration efficace de L-arginine est d'au moins 5 % en poids / volume d'excipient d'administration.

4. Procédé selon la revendication 1, dans lequel l'excipient d'administration comprend en outre un ou plusieurs éléments parmi de l'eau, de l'huile minérale, du stéarate de glycéryle, du squalène, du stéarate de propylèneglycol, de l'huile de germes de blé, du stéarate de glycéryle, du myristate d'isopropyle, du stéarate de stéryle, du polysorbate 60, du propylèneglycol, de l'acide oléique, de l'acétate de tocophérol, du collagène, du stéarate de sorbitane, de la vitamine A, de la vitamine D, de la triéthanolamine, du méthylparabène, de l'extrait d'*Aloe vera,* de l'urée d'imidazolidinyle, du propylparabène, du PND ou du BHA.

5. Procédé selon la revendication 1, dans lequel l'agent de pénétration est présent dans l'excipient d'administration dans une concentration au moins suffisante pour permettre à la L-arginine d'agir pendant au moins 3 heures.

6. Procédé selon la revendication 1, comprenant en outre un acte consistant à réappliquer l'excipient d'administration sur le sein, comprenant éventuellement la répétition de l'acte consistant à réappliquer l'excipient d'administration sur le sein entre 2 et 30 fois, inclues, pendant une période de temps d'environ 30 jours.

7. Procédé cosmétique comprenant un acte consistant à appliquer un excipient d'administration contenant de la L-arginine sur un sein, pendant une période de temps suffisante pour permettre au sein d'absorber une quantité suffisante de L-arginine pour produire une surface plus lisse sur le sein, dans lequel l'excipient d'administration comprend un agent de pénétration comprenant un sel ionique présent dans une concentration d'au moins 5 % en poids.

8. Procédé selon la revendication 7, dans lequel l'excipient d'administration comprend un ou plusieurs éléments parmi de l'eau, de l'huile minérale, du stéarate de glycéryle, du squalène, du stéarate de propylèneglycol, de l'huile de germes de blé, du stéarate de glycéryle, du myristate d'isopropyle, du stéarate de stéryle, du polysorbate 60, du propylèneglycol, de l'acide oléique, de l'acétate de tocophérol, du collagène, du stéarate de sorbitane, de la vitamine A, de la vitamine D, de la triéthanolamine, du méthylparabène, de l'extrait d'*Aloe vera,* de l'urée d'imidazolidinyle, du propylparabène, du PND ou du BHA.

9. Procédé selon la revendication 7, comprenant en outre un acte consistant à réappliquer l'excipient d'administration sur le sein, comprenant éventuellement la répétition de l'acte consistant à réappliquer l'excipient d'administration sur le sein après entre environ 8 heures et environ 48 heures après l'acte consistant à appliquer l'excipient d'administration.

10. Procédé selon la revendication 1 ou la revendication 7, dans lequel l'excipient d'administration est une crème.

11. Procédé selon la revendication 1 ou la revendication 7, comprenant l'étape consistant à faire pénétrer l'excipient d'administration dans le sein par massage.

12. Procédé selon la revendication 1 ou la revendication 7, dans lequel le sel ionique comprend un ou plusieurs éléments parmi du chlorure de lithium, du chlorure de sodium, du chlorure de potassium, du chlorure de calcium, du chlorure de magnésium, ou du chlorure de choline.

13. Procédé selon la revendication 1 ou la revendication 7, dans lequel le sel ionique est présent à une concentration d'au moins environ 10 % en poids.
